Europäisches Patentamt

(19) **European Patent Office**

**Office européen des brevets**

(11) Numéro de publication: **0 152 730 B1**

(12) **FASCICULE DE BREVET EUROPEEN**

(45) Date de publication du fascicule du brevet:
13.05.87

(51) Int. Cl.⁴: **C 12 P 5/02, C 12 M 1/00, C 02 F 3/34**

(21) Numéro de dépôt: **84870175.1**

(22) Date de dépôt: **13.12.84**

(54) **Procédé de production de méthane par digestion anaerobie de matières organiques.**

(30) Priorité: **15.12.83 LU 85141**

(43) Date de publication de la demande:
**28.08.85 Bulletin 85/35**

(45) Mention de la délivrance du brevet:
**13.05.87 Bulletin 87/20**

(84) Etats contractants désignés:
**AT BE CH DE FR GB IT LI LU NL SE**

(56) Documents cités:
**EP - A - 0 036 065**
**EP - A - 0 037 612**
**FR - A - 2 443 504**
**US - A - 4 022 665**

**PROGRESS IN ENERGY AND COMBUSTION SCIENCE, vol. 8, no. 2, 1982, pages 135-138, Oxford, GB; P.N. HOBSON et al.: "Production and use of biogas in agriculture"**

(73) Titulaire: **L'Etat belge, représenté par le Secrétaire Général des Services de la Programmation de la Politique Scientifique, 8 rue de la Science, B-1040 Bruxelles (BE)**

(72) Inventeur: **Legros, Alain, 11, rue Docteur Maître, B-6248 Nalinnes (BE)**
Inventeur: **Nyns, Edmond-Jacques, 2, Tienne du Chenois, B-1302 Dion-Valmont (BE)**
Inventeur: **Naveau, Henry, 38, rue de l'Institut, B-1330 Rixensart (BE)**

(74) Mandataire: **De Brabanter, Maurice et al, Bureau VANDER HAEGHEN 63 Avenue de la Toison d'Or, B-1060 Bruxelles (BE)**

## Description

La présente invention est relative à un procédé de biométhanisation de substrats organiques solides ou semi-solides, dans lequel on soumet un tel substrat mis en suspension dans de l'eau à l'action de communautés de microorganismes ayant successivement pour effet d'extraire du substrat des produits hydrosolubles, de transformer ces produits solubilisés en acides et/ou alcools et de méthaniser ces acides et/ou alcools dans deux fermenteurs montés en série.

La biométhanisation de matières organiques solides ou semi-solides est un processus connu qui utilise des communautés de microorganismes travaillant en symbiose par digestion anaérobie des fractions biodégradables de ces matières organiques.

Une première communauté de microorganismes attaque la matière organique solide ou semi-solide et transforme cette matière organique en produits hydrosolubles, tels que des sucres, des lipides, des acides aminés, des protéines, etc.... Ces produits solubilisés sont ensuite transformés, par des communautés de microorganismes bactériens, en acides gras volatils (tels que acides acétique, propionique et butyrique) et autres produits (tels que lactate, éthanol, bicarbonate, hydrogène, etc.) qui sont à leur tour transformés en acide acétique, hydrogène et bicarbonate. Enfin, une dernière communauté bactérienne transforme l'acide acétique, l'hydrogène et le bicarbonate en un mélange de méthane et d'oxyde de carbone appelé «biogaz».

Il est connu de produire un tel biogaz par digestion de substrats organiques solides ou semi-solides dans un seul fermenteur, mais le faible taux de croissance des bactéries méthanigènes nécessite de long temps de séjour du substrat dans le fermenteur, de l'ordre de 15 à 30 jours, et donc l'emploi de grands volumes de fermentation.

On connaît également un procédé de biométhanisation de substrats organiques solides et/ou semi-solides en deux étapes (Ghosh et al, Journal WPCF, 47, (1), pages 30-45). Dans ce procédé, on utilise un premier fermenteur, dont le contenu est agité en permanence et dans lequel s'opère, à un pH généralement compris entre 4 et 6, l'extraction des matières solubles et leur transformation en acides gras volatils. Après ces traitements, tout le contenu du premier fermenteur est transvasé dans un second fermenteur, dans lequel s'opère la méthanisation des acides gras. Ce procédé en deux étapes a l'inconvénient d'être lent, tandis qu'il nécessite l'emploi de deux fermenteurs de grande capacité.

On connaît aussi un autre procédé de biométhanisation de matières organiques solides ou semi-solides en deux étapes (Rijkens, Energy from Biomass, Serie E, édité par Reidel D Publ. Co, Dordrecht, Pays-Bas, 1981, 1, 121-125). Dans ce procédé, les réactions aboutissant à la formation d'acides s'effectuent dans un premier fermenteur du type «batch», dont la phase liquide est amenée dans un digesteur du type «up flow» dans lequel s'accumule de la biomasse et dont l'effluent liquide est utilisé comme liquide de dilution du contenu du fermenteur «batch». Lorsque la production d'acides gras volatils est terminée dans le fermenteur «batch», celui-ci est déconnecté du digesteur «up-flow» et ce dernier est reconnecté lui-même à und second fermenteur «batch», tandis que le premier fermenteur «batch» devient alors un fermenteur de méthanisation des acides gras volatils. Ce procédé nécessite l'emploi d'une série de fermenteurs «batch» pour un seul réacteur «up-flow».

On connaît aussi par le brevet U.S.A. 4 022 665 un procédé de digestion anaérobie de matières organiques en deux étapes. Dans la première étape, qui s'opère dans un premier digesteur, la matière organique est liquéfiée et décomposée en acides de faible poids moléculaire et autre produits à un pH de 5 à 6, tandis que dans la seconde phase, qui s'opère dans un second digesteur, dans lequel est transvasé le contenu du premier digesteur, l'effluent de ce dernier est méthanisé à un pH de 6,8 à 7,4. Dans ce procédé connu, il ne se forme pratiquement pas de méthane dans le premier digesteur (voir tableau II; colonne 6). Un inconvénient de ce procédé réside dans le fait que la quantité de matière de départ introduite dans le premier digesteur doit être réglée, de manière à éviter une acidification trop importante du contenu de ce premier digesteur, une telle acidification pouvant diminuer le pH jusqu'à une valeur inhibant les réactions de solubilisation et d'acidogénèse. Pour éviter cet inconvénient, on prévoit, dans le procédé selon le brevet U.S.A. 4 022 665, d'hydrolyser la matière organique de départ dans une zone de préhydrolyse et d'ajouter un agent neutralisant à cette matière organique hydrolysée avant son introduction dans le premier digesteur.

Enfin, on connaît par le brevet français FR-A-2 443 504 un procédé de biométhanisation, dans lequel un substrat organique (pulpes et déchets de betteraves) est chargé dans un premier digesteur, dans lequel ledit substrat est simplement liquéfié par hydrolyse enzymatique pour former une masse contenant des produits solubles (glucides), cette masse étant envoyée, dans sa totalité, dans un second digesteur où elle est soumise à une acidogénèse et à une méthanisation par perfusion à travers une biomasse anaérobie. De ce second digesteur est soutirée, après filtration dans un séparateur, une phase liquide contenant des enzymes hydrolysants qui est renvoyée dans le premier digesteur où elle assure la liquéfaction du substrat organique. La biométhanisation incomplète du substrat dans le second fermenteur ou digesteur provoque une accumulation d'acides gras volatils dans la phase liquide du système, en sorte que ce second digesteur doit être soumis périodiquement à des purges de déconcentration, entraînant des pertes d'acides gras méthanisables.

La présente invention a pour objet un procédé du type défini dans le premier paragraphe du présent mémoire qui offre l'avantage, par rapport au procédé du brevet français FR-A-2 443 504 de ne pas nécessiter des purges entraînant des pertes de produits encore méthanisables.

Le procédé suivant l'invention est basé sur l'idée de réaliser l'hydrolyse (extraction de matières solubles d'un substrat organique solide ou semi-solide), l'acidogénèse et la méthanisation dans un seul et même fermenteur, dont le contenu est maintenu à un pH approprié, et de méthaniser dans un second fermenteur l'excès d'acides et/ou d'alcools produits dans le premier fermenteur, ce qui a pour effet, non seulement d'accélérer la production de biogaz et d'en augmenter le rendement, mais également d'empêcher l'accumulation d'acides gras volatils (nécessitant des purges périodiques) échappant à la méthanisation.

Le procédé suivant l'invention ne nécessite que des purges périodiques de produits qui ne sont plus méthanisables, de sorte que son rendement est supérieur à celui du procédé connu par le FR-A-2 443 504. Au surplus, le procédé suivant l'invention ne nécessite pas le passage de la totalité du substrat dans le second digesteur, seul l'excès d'acides gras volatils produit dans le premier digesteur devant être amené dans le second digesteur.

La présente invention a aussi pour objet un procédé du type défini dans le premier paragraphe du présente mémoire, qui présente l'avantage, par rapport au procédé décrit dans le brevet U.S.A. 4 022 665, de ne pas nécessiter de préhydrolyse de la matière organique de départ, ni l'addition d'un agent neutralisant à cette matière organique hydrolysée, étant donné que, dans le procédé suivant l'invention, on extrait du premier digesteur les acides formés avant qu'ils y atteignent des concentrations entraînant une diminution du pH susceptible d'inhiber les réactions de solubilisation, d'acidification et de méthanisation dans le premier digesteur.

Le procédé suivant l'invention du type défini dans le premier paragraphe du présent mémoire est essentiellement caractérisé en ce qu'on soumet la suspension de substrat organique chargée dans le premier fermenteur à une solubilisation des matières organiques y contenues, à une acidogénèse et à une méthanisation, en y maintenant un pH de 6,5 à 8,5, en ce qu'on soutire du premier fermenteur un effluent liquide dépourvu de matières solides et contenant l'excès d'acides et/ou d'alcools non méthanisés et on envoie cet effluent liquide dans le second fermenteur chargé de microorganismes méthanigènes, où cet effluent liquide est méthanisé également à un pH de 6,5 à 8,5 au contact de microorganismes méthanigènes, et en ce qu'on soutire du second fermenteur un effluent liquide neutre ou alcalin chargé de microorganismes méthanigènes et on recycle, éventuellement de manière discontinue, cet effluent liquide neutre ou alcalin dans le premier fermenteur.

L'excès d'acides et/ou d'alcools, auquel il est fait référence ci-dessus, est la quantité de ces produits qui, si elle n'était pas soutirée du premier digesteur et envoyée dans le second digesteur, inhiberait partiellement ou ralentirait dans le premier digesteur la réaction de méthanisation. En effet, la réaction de méthanisation ne peut pas se produire à un pH inférieur à 6,5; la solubilisation peut se produire à un pH inférieur, mais elle est alors partiellement inhibée ou ralentie.

Le procédé suivant l'invention permet d'obtenir des quantités substantielles de méthane dans les deux fermenteurs monté en série.

Selon une particularité du procédé suivant l'invention, on introduit périodiquement de nouvelles charges de substrat organique en suspension dans de l'eau dans le premier fermenteur et on évacue périodiquement une fraction de liquide neutre ou alcalin soutirée du second fermenteur, cette fraction correspondant sensiblement à la quantité de liquide introduite dans le premier fermenteur avec chaque nouvelle charge de substrat.

Dans une forme de réalisation avantageuse du procédé suivant l'invention, on interrompt le soutirage de l'excès d'acides et/ou d'alcools du premier fermenteur lors de l'introduction de chaque nouvelle charge de substrat organique dans ce premier fermenteur et lors de chaque soutirage de liqueur mixte de ce premier fermenteur.

On utilise, de préférence, comme premier fermenteur, un récipient d'une capacité supérieur à celle du récipient utilisé comme second fermenteur, le rapport de la capacité du premier fermenteur à celle du second fermenteur étant avantageusement compris entre 2,5 et 10.

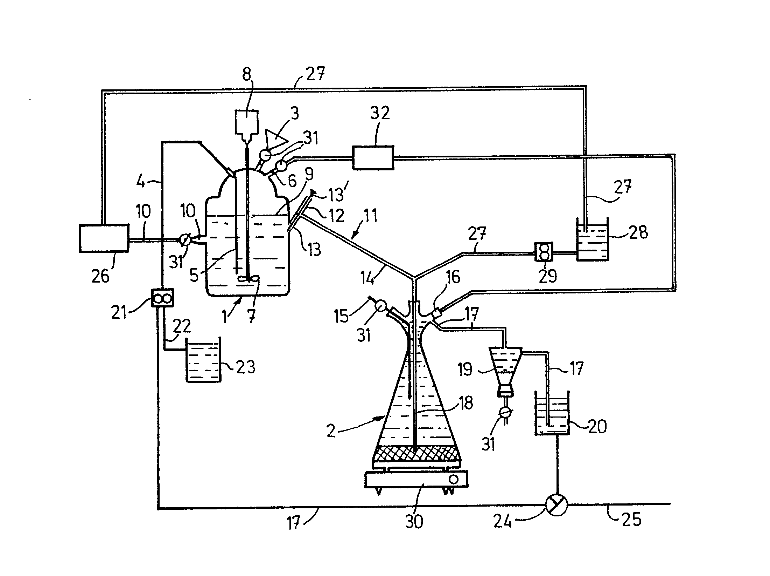

D'autres particularités et détails de procédé suivant l'invention ressortiront de la description suivante dans laquelle il est fait référence au dessin ci-annexé, dont la figure unique montre schématiquement et à titre d'exemple une installation dans laquelle le procédé suivant l'invention peut être mis en œuvre.

Le dessin ci-annexé montre une installation comprenant deux fermenteurs 1 et 2 montés en série. Le premier fermenteur 1 est équipé d'une entrée 3 servant à y introduire des charges de matières organiques solides ou semi-solides biodégradables, telles que des algues, des ordures, des résidus d'égoûts, etc... en suspension dans de l'eau. Un conduit 4 se terminant par un tube 5 plongeant dans le premier fermenteur 1 sert à y recycler un effluent du second fermenteur 2, comme décrit plus loin. Le fermenteur 1 présente une sortie 6 permettant d'en évacuer le biogaz qui s'y forme, ce biogaz étant recueilli dans un collecteur schématisé en 32. Le fermenteur 1 est également équipé d'un agitateur 7 actionné par un moteur 8. A un niveau situé entre celui de l'extrémité inférieure libre du tube plongeur 5 et le niveau libre 9 de la suspension aqueuse de substrat organique contenu dans le premier fermenteur 1, ce dernier est muni d'un conduit de soutirage 10 par lequel une liqueur aqueuse contenant les factions non biodégradables éventuellement contenues dans les charges de substrat du premier fermenteur sont soutirées comme décrit plus loin. Enfin, un conduit 11, en forme de T incliné, débouche par une branche 12 dans le premier fermenteur, la branche 12 pouvant être obturée par un bouchon 13 actionné par une tige 13'. Le conduit en T 11 est relié par une autre branche 14 au second fermenteur 2.

Le second fermenteur 2, dont la capacité est largement inférieure à celle du premier fermenteur 1 est muni d'une sortie 15 permettant d'échantillonner la liqueur du fermenteur, d'une sortie 16 permettant d'en évacuer le biogaz qui s'y forme, et pouvant être reliée au collecteur de biogaz 32, d'un conduit 17 débouchant dans la partie supérieure du second fermenteur 2 pour le soutirage d'un effluent liquide comme expliqué plus loin et d'un tube plongeant 18 raccordé à la branche 14 du conduit en T 11.

Dans le conduit 17 sont éventuellement insérés un décanteur 19 et un réservoir de stockage 20. Ce conduit 17 est relié au conduit 4 éventuellement par l'intermédiaire d'une pompe doseuse 21 qui peut être éventuellement alimentée en effluent liquide du second fermenteur 2 par un conduit 22 relié à un réservoir de stockage 23. Dans le conduit 17 est également insérée une vanne à trois voies 24 permettant l'évacuation d'une partie de l'effluent liquide du second fermenteur par un conduit d'évacuation 25.

L'installation comporte encore un filtre 26 auquel aboutit le conduit de soutirage 10 du premier fermenteur 1, ainsi qu'un conduit 27 pour amener le filtrat liquide obtenu dans le filtre 26 dans le conduit 11, un réservoir de stockage 28 et une pompe doseuse 29 étant éventuellement insérés dans le conduit 27.

Le second fermenteur 2 peut être équipé d'un agitateur, tel qu'un agitateur magnétique 30.

Des vannes 31 sont prévues sur les différents conduits montrés sur le dessin.

Le procédé suivant l'invention est décrit ci-après en se référant à l'installation décrite ci-dessus.

Dans le fermenteur 1 contenant une biomasse de communautés de microorganismes permettant la liquéfaction, l'acidogénèse et la méthanogénèse, un substrat organique solide ou semi-solide en suspension aqueuse est introduit par l'entrée 3. Après agitation de courte durée du contenu du fermenteur 1, on laisse les réactions de liquéfaction, d'acidogénèse et de méthanisation se dérouler dans le fermenteur 1, à un pH de 6,5 à 8,5 de préférence de 7 à 7,5, sans que son contenu soit encore mélangé à l'aide de l'agitateur 7. De ce fermenteur 1, on soutire ensuite de manière continue un effluent liquide dépourvu de matières solides et contenant l'excès d'acides et/ou d'alcools non méthanisés dans le fermenteur 1. Cet effluent liquide est soutiré par trop-plein ou débordement, à travers le conduit en T 11. Le conduit 11 amène par sa branche 14 et le tube plongeant 18, l'effluent liquide contenant l'excès d'acides et/ou d'alcools dans le fermenteur 2, où il est méthanisé au contact d'une biomasse constituée d'une communauté de bactéries méthanigènes préalablement introduite, à un pH de 6,5 à 8,5, de préférence de 7 à 7,5.

Le biogaz formé dans le fermenteur 1 est extrait de celui-ci par sa sortie 6 et recueilli dans le collecteur 32. Le biogaz formé dans le fermenteur 2 en est extrait par sa sortie 16 et peut être amené dans le collecteur 32. En somme, le fermenteur 2 sert à méthaniser l'excès d'acides et/ou d'alcools qui n'a pas été méthanisé dans le fermenteur 1 et qui doit en être soutiré pour y maintenir le pH approprié. Ce fermenteur 2 ne contient sensiblement pas de substrat solide, mais uniquement une fraction liquide contenant ledit excès d'acides et/ou d'alcools. A la partie supérieure du fermenteur 2 est soutirée par trop-plein ou débordement, dans le conduit 17, un effluent liquide neutre ou alcalin ne contenant sensiblement plus de composants méthanisables. Cet effluent neutre ou alcalin est éventuellement décanté dans le décanteur 19 et stocké dans le réservoir de stockage 17 relié au conduit 4 éventuellement par l'intermédiaire de la pompe doseuse 21. En cas de besoin, l'effluent liquide neutre ou alcalin, est recueilli, avant son recyclage dans le fermenteur 1, dans le réservoir 23, d'où il est amené par le conduit 22 dans la pompe de recyclage 21 qui sert à le recycler dans le fermenteur 1 par le tube plongeant 5 dont est équipé ce fermenteur 1. L'effluent liquide neutre ou alcalin recyclé dans le fermenteur 1 sert à y régler le pH à une valeur de 6,5 à 8,5, de préférence de 7 à 7,5, propice à y favoriser les réactions de liquéfaction, d'acidogénèse et de méthanisation du substrat dans le fermenteur 1.

Lorsque le substrat a séjourné dans le fermenteur 1 pendant un temps suffisant pour que la majeure partie des matières organiques biodégradables qu'il contient aient été liquéfiées, acidifiées et méthanisées, on soutire de ce fermenteur 1 une liqueur mixte composée en majeure partie de matière non biodégradable en suspension aqueuse. Cette liqueur mixte soutirée par le conduit 10 est filtrée dans le filtre 26, dans lequel la matière non biodégradable en est séparée pour être évacuée et dont le filtrat liquide est amené par le conduit 27 dans le conduit 14 alimentant le fermenteur 2 en liquide acide, lorsque ce filtrat contient encore des acides organiques méthanisables. En cas de besoin, le filtrat liquide obtenu dans le filtre 26 est stocké dans le réservoir de stockage 28 et amené, par la pompe doseuse 29, dans le conduit d'alimentation 11 du second fermenteur 2.

L'alimentation du fermenteur 1 en charges du substrat organique solide ou semi-solide s'effectue de matière à maintenir dans ce fermenteur un volume sensiblement constant de suspension de ce substrat, en tenant compte du soutirage périodique par le conduit 10 de liqueur mixte, du recyclage d'effluent neutre ou alcalin provenant du fermenteur 2 dans le fermenteur 1 par le tube plongeant 5 et du soutirage de l'excès d'acide et/ou d'alcool du fermenteur 1 par le conduit en T 11. Il se produit ainsi dans le fermenteur 1 une formation continue de biogaz, tandis qu'une formation de biogaz s'effectue aussi pratiquement en continu dans le fermenteur 2.

En cas de besoin, une partie de l'effluent liquide neutre ou alcalin soutiré du fermenteur 2 est évacué par le conduit d'évacuation 25 en manoeuvrant la vanne à trois voies 24.

On voit ainsi que le procédé suivant l'invention assure une méthanisation complète des matières biodégradables contenues dans le substrat organique de départ, sans qu'une fraction de ces matières biodégradables puisse échapper à cette méthanisation. Ainsi, le rendement du procédé est optimal, le fermenteur 2 servant à transformer en biogaz l'excès des acides et/ou alcools de faible poids moléculaire qui n'ont pas été méthanisés dans le fermenteur 1 et qui sont récupérés dans la liqueur mixte soutirée du fermenteur 1 et surtout dans la fraction liquide dépourvue de matières solides, qui est soutirée par le conduit T du fermenteur 1.

Le procédé suivant l'invention est illustré par les exemples suivants.

*Exemple 1*

On a utilisé comme substrat des algues Hydrodictyon de composition suivante:

matières sèches totales (TS): 939/g/kg
matières volatiles (VS): 482/g/kg
demande chimique en oxygène
(DCO): 640 g $O_2$/kg.

Dans le premier fermenteur 1 d'une capacité de 2,8 litres contenant des communautés de bactéries liquéfiantes, acidifiantes et méthanigènes, on a introduit des charges d'algues à raison d'une alimentation par jour, de façon à assurer dans le fermenteur 1 les conditions suivantes:

- temps de séjour moyen hydraulique ($\Theta$H): 1,04 jour
- temps de séjour moyen des solides ($\Theta$S): 5,0 jours
- charge volumique ($B_V$): 16 g DCO/litre/jour
- pH du fermenteur 1: 7,2.

L'alimentation continue en liquide contenant l'excès d'acides et/ou d'alcools (pH: 7,2-7,5) du fermenteur 2 d'une capacité de 0,9 litre et le soutirage d'effluent neutre ou alcalin (pH: 7,5-8) de ce fermenteur (cet effluent neutre ou alcalin étant recyclé dans le fermenteur 1) ont eu lieu de manière à assurer les conditions suivantes dans le fermenteur 2:

- temps de séjour moyen hydraulique ($\Theta$H): 0,3 jour
- charge volumique ($B_V$): 7,41 g DCO/litre/jour
- pH: 7,5.

Le contenu des deux fermenteurs 1 et 2 a été maintenu à une température de 35°C ± 1°C. La charge globale de l'installation a été de 12,5 g DCO/litre/jour et le temps de séjour de chaque fraction d'algues de 5 jours.

La production de biogaz globale obtenue en régime a été de 3,39 l de gaz par litre de volume de matière fermentescible et par jour avec une concentration moyenne en méthane de 66%. Cette production de biogaz s'est répartie de la manière suivante: 3,78 litres de biogaz (65% $CH_4$) par litre et par jour dans le premier fermenteur 1 et 1,97 litre de biogaz (75% $CH_4$) par litre et par jour dans le second fermenteur 2. Le rendement en méthane obtenu était de 0,18 l de $CH_4$/g de DCO.

*Exemple 2*

On a utilisé comme substrat une fraction organique de résidus urbains de composition suivante:

- matières sèches totales (TS): 247 g/kg
- matières volatiles (Vs): 204 g/kg
- demanche chimique en oxygène
  (DCO): 352 g/kg

Dans le premier fermenteur 1 d'une capacité de 2,8 litres contenant des communautés de bactéries liquéfiantes, acidifiantes et méthanigènes, on a introduit des charges de la fraction organique de résidus urbains à raison d'une alimentation par jour, de façon à assurer dans le fermenteur 1 les conditions suivantes:

- temps de séjour moyen hydraulique ($\Theta$H): 1,4 jour
- temps de séjour moyen des solides ($\Theta$S): 7 jours
- charge volumique ($B_V$): 23,35 g de DCO/litre/jour
- pH du fermenteur 1: 7,3

L'alimentation continue en liquide contenant l'excès d'acides et/ou d'alcools du fermenteur 2 d'une capacité de 0,9 litre et le soutirage d'effluent neutre ou alcalin de ce fermenteur 2 (cet effluent neutre ou alcalin étant recyclé dans le fermenteur 1) ont eu lieu de manière à assurer les conditions suivantes dans le fermenteur 2:

- temps de séjour moyen hydraulique ($\Theta$H): 0,4 jour
- charge volumique ($B_V$): 24,36 g de DCO/litre/jour
- pH: 7,5
- agitation: 2 secondes par heure.

Le contenu des deux fermenteurs 1 et 2 a été maintenu à une température de 35°C ± 1°C. La charge globale de l'installation a été de 18,17 g de DCO/litre/jour et le temps de séjour de chaque charge de résidus organiques urbains de 7 jours.

En régime, on a obtenu 4,95 litres de biogaz par litre de volume de matière fermentescible et par jour avec une teneur moyenne en méthane de 68%.

Cette production de biogaz s'est répartie de la manière suivante: 4,74 litres de biogaz (65% de $CH_4$) par litre et par jour dans le premier fermenteur 1 et 5,60 litres de biogaz (78% de $CH_4$) par litre et par jour dans le second fermenteur 2. Le rendement en méthane a donc été dans ce cas de 0,18 litre de $CH_4$/g de DCO.

Le procédé suivant l'invention offre principalement l'avantage par rapport aux procédés connus de permettre une augmentation sensible de la charge volumique globale d'une installation ne comportant que deux fermenteurs et une diminution spectaculaire du temps de séjour du substrat organique par rapport aux procédés connus dans lesquels la méthanisation de substrats organiques s'effectue dans des fermenteurs classiques de type mélangé.

Un autre avantage du procédé suivant l'invention est de permettre une transformation optimale en biogaz des matières biodégradables contenues dans les substrats organiques, sans perte notable de ces matières biodégradables, à l'inverse des procédés connus dans lesquels se produit inévitablement une méthanisation incomplète des matières biodégradables.

Dans les procédés connus, la biométhanisation n'est en tout cas pas complète dans le cas d'une surcharge entraînant une méthanisation partielle des acides gras volatils formés ou dans le cas d'un temps de séjour trop faible entraînant une liquéfaction et une acidogénèse incomplètes.

Ces avantages sont rendus possibles essentiellement grâce au maintien d'un pH optimal de liquéfaction (6,5 à 8,5, de préférence 7,0 à 7,5) dans le fermenteur 1, par le recyclage de l'effluent neutre ou alcalin (effluent biocarbonaté) du fermenteur 2 dans le fermenteur 1, grâce au soutirage continu du seul excès d'acides et/ou d'alcools non méthanisés dans le premier fermenteur 1, ces produits étant méthanisés dans le second fermenteur, et grâce au recyclage de microorganismes méthanigènes avec l'effluent neutre ou alcalin dans le fermenteur 1 à partir du fermenteur 2.

La forme des fermenteurs 1 et 2 peut être variable.

Dans le fermenteur 1, le temps de séjour de la fraction solide (substrat organique) peut varier entre 3 et 25 jours et même davantage, en fonction de la vitesse de biodégradabilité du substrat. Dans ce même fermenteur 1, le temps de séjour hydraulique peut être de 10 à 0,3 jours, en fonction de la vitesse d'acidogénèse du substrat. La charge volumique du premier fermenteur peut également varier par exemple entre 1 et 25 DCO/litre/jour.

Au lieu de soutirer les effluent liquides des fermenteurs 1 et 2 par débordement ou trop-plein, on peut effectuer ces soutirages à l'aide de pompes.

Dans le second fermenteur 2, le temps de séjour hydraulique est également variable et peut être compris entre 0,1 et 10 jours, tandis que la charge volumique peut être de 1 à 30 g de DCO/litre/jour.

Dans le second fermenteur 2, la biomasse, c'est-à-dire la masse de bactéries méthanigènes, peut être fixée sur un support ou être libre, tandis que l'agitation intermittente du contenu de ce second fermenteur est facultative.

L'effluent du second fermenteur peut être décanté ou non, avant son recyclage dans le premier fermenteur. En cas de décantation, la biomasse décantée peut être renvoyée, en discontinu, dans le premier fermenteur 1.

Avant son recyclage dans le premier fermenteur, l'effluent liquide du second fermenteur peut être éventuellement stocké et ses caractéristiques physico-chimiques peuvent être éventuellement modifiées.

La liqueur mixte soutirée du premier fermenteur 1 est avantageusement filtrée, pour éliminer la matière non dégradable du substrat et, si le filtrat liquide contient encore des matières méthanisables, cette phase liquide est avantageusement envoyé dans le second fermenteur 2.

Si l'effluent liquide du premier fermenteur 1 est soutiré au moyen d'une pompe, les deux fermenteurs 1 et 2 peuvent être évidemment à un même niveau.

La séparation et le soutirage de l'effluent liquide du premier fermenteur 1 s'effectuent avantageusement en n'agitant pas le contenu de ce fermenteur 1 et en profitant ainsi de la flottation et de le décantation des particules solides. Cependant, on peut placer n'importe quel système de séparation liquide/solide à la sortie de l'effluent liquide du premier fermenteur, dont le contenu peut alors être éventuellement agité, de préférence de manière discontinue.

**Revendications**

1. Procédé de biométhanisation de substrats organiques solides ou semi-solides, dans lequel on soumet un tel substrat mis en suspension dans de l'eau à l'action de communautés de microorganismes ayant successivement pour effet d'extraire du substrat des produits hydrosolubles, de transformer ces produits solubilisés en acides et/ou alcools et de méthaniser ces acides et/ou alcools dans deux fermenteurs montés en série, caractérisé en ce qu'on soumet la suspension de substrat organique chargée dans le premier fermenteur (1) à une solubilisation des matières organiques y contenues, à une acidogénèse et à une méthanisation, en y maintenant un pH de 6,5 à 8,5, en ce qu'on soutire du premier fermenteur (1) un effluent liquide dépourvu de matières solides et contenant l'excès d'acides et/ou d'alcools non méthanisés et on envoie cet effluent liquide dans le second fermenteur (2) chargé de microorganismes méthanigènes, où cet effluent liquide est méthanisé également à un pH de 6,5 à 8,5 au contact des microorganismes méthanigènes, et en ce qu'on soutire du second fermenteur (2) un effluent liquide neutre ou alcalin chargé de microorganismes méthanigènes et on recycle, éventuellement de manière discontinue, cet effluent liquide neutre ou alcalin dans le premier fermenteur.

2. Procédé suivant la revendication 1, caractérisé en ce qu'on soutire périodiquement du premier fermenteur (1) une liqueur mixte contenant de la matière solide ou semi-solide non biodégradable.

3. Procédé suivant la revendication 2, caractérisé en ce qu'on filtre la liqueur mixte précitée pour en séparer la matière solide ou semi-solide non biodégradable et on recycle le filtrat obtenu dans le second fermenteur (2) lorsque ce filtrat contient encore des matières méthanisables.

4. Procédé suivant l'une quelconque des revendications précédentes, caractérisé en ce qu'on introduit prériodiquement de nouvelles charges de substrat organique en suspension dans de l'eau dans le premier fermenteur (1) et on évacue périodiquement une fraction de liquide neutre ou alcalin soutirée du second fermenteur (2), cette fraction correspondant sensiblement à la quantité de liquide introduite dans le premier fermenteur (1) avec chaque nouvelle charge de substrat.

5. Procédé suivant l'une quelconque des revendications précédentes, caractérisé en ce qu'on homogénéise le contenu du premier fermenteur (1) chaque fois que l'on y introduit une nouvelle charge de substrat organique et qu'on en soutire la liqueur mixte précitée.

6. Procédé suivant l'une quelconque des revendications précédentes, caractérisé en ce qu'on interrompt le soutirage de l'excès d'acides et/ou d'alcools du premier fermenteur (1) lors de l'introduction de chaque nouvelle charge de substrat organique dans ce premier fermenteur (1) et lors de chaque soutirage de liqueur mixte de ce premier fermenteur (1).

7. Procédé suivant l'une quelconque des revendications précédentes, caractérisé en ce qu'on utilise comme premier fermenteur (1) un récipient d'une capacité supérieure à celle du récipient utilisé comme second fermenteur (2).

8. Procédé suivant la revendication 7, caractérisé en ce que le rapport de la capacité du premier fermenteur (1) à celle du second fermenteur (2) est compris entre 2,5 et 10.

9. Procédé suivant la revendication 1 ou 8, caractérisé en ce qu'on soumet l'effluent liquide neutre ou alcalin soutiré du second fermenteur (2) à une décantation et on renvoie, en discontinu, dans le premier fermenteur (1) la masse de miocroorganismes méthanigènes séparée par décantation.

**Patentansprüche**

1. Biomethanisierungsverfahren von festen oder halbfesten organischen Substraten, in welchem man ein solches Substrat in Suspension in Wasser der Einwirkung von Mikroorganismenkulturen unterwirft, um aufeinanderfolgend vom Substrat wasserlösliche Produkte zu extrahieren, diese löslich gemachten Produkte in Säuren und/oder Alkohole überzuführen und diese Säuren und/oder Alkohole in zwei hintereinander angeordneten Fermentern zu methanisieren, dadurch gekennzeichnet, dass man die in den ersten Fermenter (1) eingebrachte Suspension des organischen Substrats einer Löslichmachung von darin enthaltenen organischen Materialien, einer Azidogenese und einer Methanisierung unterwirft, wobei man einen pH von 6,5 bis 8,5 aufrechterhält, und dass man vom ersten Fermenter (1) einen flüssigen Abfluss entfernt, der keine festen Materialien aufweist und den Überschuss der nicht methanisierten Säuren und/oder Alkohole enthält, und diesen flüssigen Abfluss in den zweiten Fermenter (2) leitet, der mit methanerzeugenden Mikroorganismen beladen ist, wo dieser flüssige Abfluss in Berührung mit den methanerzeugenden Mikroorganismen ebenfalls bei einem pH von 6,5 bis 8,5 methanisiert wird, und dass man vom zweiten Fermenter (2) einen neutralen oder alkalischen flüssigen Abfluss, der mit methanerzeugenden Mikroorganismen beladen ist, entfernt und diesen flüssigen Abfluss, gegebenenfalls auf diskontinuierliche Weise, in den ersten Fermenter rückführt.

2. Verfahren nach Anspruch 1, dadurch gekennzeichnet, dass man vom ersten Fermenter (1) eine Mischflüssigkeit, die das nicht-bioabbaubare feste oder halbfeste Material enthält, periodisch entfernt.

3. Verfahren nach Anspruch 2, dadurch gekennzeichnet, dass man die genannte Mischflüssigkeit zum Abtrennen von nichtbioabbaubarem festen oder halbfesten Material filtriert und das erhaltene Filtrat in den zweiten Fermenter (2) führt, wenn dieses Filtrat noch methanisierbare Materialien enthält.

4. Verfahren nach einem der vorhergehenden Ansprüche, dadurch gekennzeichnet, dass man in den ersten Fermenter (1) periodisch neue Chargen von organischem Substrat in Suspension in Wasser einführt und periodisch eine Fraktion von vom zweiten Fermenter (2) entfernter neutraler oder alkalischer Flüssigkeit ableitet, wobei diese Fraktion im wesentlichen dem in den ersten Fermenter (1) mit jeder neuen Substratcharge eingebrachten Flüssigkeitsanteil entspricht.

5. Verfahren nach einem der vorhergehenden Ansprüche, dadurch gekennzeichnet, dass man den Inhalt des ersten Fermenters (1) jedesmal, wenn man eine neue Charge von organischem Substrat einführt und wenn man die obgenannte Mischflüssigkeit entfernt, homogenisiert.

6. Verfahren nach einem der vorhergehenden Ansprüche, dadurch gekennzeichnet, dass man die Entfernung des Überschusses von Säuren und/oder Alkoholen vom ersten Fermenter (1) bei der Einführung jeder neuen Charge von organischem Substrat in diesen ersten Fermenter (1) und bei jeder Entfernung von Mischflüssigkeit von diesem ersten Fermenter (1) unterbricht.

7. Verfahren nach einem der vorhergehenden Ansprüche, dadurch gekennzeichnet, dass man als ersten Fermenter (1) einen Behälter mit einer grösseren Kapazität als jene des als zweiter Fermenter (2) verwendeten Behälters verwendet.

8. Verfahren nach Anspruch 7, dadurch gekennzeichnet, dass das Verhältnis der Kapazität des ersten Fermenters (1) zu jener des zweiten Fermenters (2) 2,5 bis 10 beträgt.

9. Verfahren nach Anspruch 1 oder 8, dadurch gekennzeichnet, dass man den flüssigen neutralen oder alkalischen Abfluss, der vom zweiten Fermenter (2) entfernt wird, dekantiert und die abdekantierte Masse der methanerzeugenden Mikroorganismen diskontinuierlich in den ersten Fermenter (1) rückführt.

**Claims**

1. Process for the biomethanization of solid or semi-solid organic substrates, in which such a substrate suspended in water is subjected to the action of communities of microorganisms, having the effect of successively extracting water soluble products from the substrate, converting these solubilized products into acids and/or alcohols and methanizing these acids and/or alcohols in two fermentors mounted in series, characterised in that the suspension of organic substrate introduced into the first fermentor (1) is subjected to a solubilization of the organic substances contained therein, to an acidogenesis and to a methanization while the pH is maintained at 6.5 to 8.5, in that a liquid effluent deprived of solid substances and containing the excess of non-methanized acids and/or alcohols is withdrawn from the first fermentor (1) and this liquid effluent is transferred to the second fermentor (2) which is charged with methanogenic microorganisms, in which second fermentor this effluent liquid is methanized, also at a pH of 6.5 to 8.5, in contact with the methanogenic microorganisms, and in that a neutral or alkaline effluent liquid charged with methanogenic microorganisms is withdrawn from the second fermentor (2), and this effluent neutral or alkaline liquid is recycled to the first fermentor, optionally discontinuously.

2. Process according to claim 1, characterised in that a mixed liquor containing non-biodegradable solid or semi-solid substance is periodically withdrawn from the first fermentor (1).

7

3. Process according to claim 2, characterised in that the aforesaid mixed liquor is filtered to separate the non-biodegradable solid or semi-solid substance and the filtrate obtained is recycled to the second fermentor (2) when this filtrate still contains methanizable substances.

4. Process according to any one of the preceding claims, characterised in that fresh batches of organic substrate suspended in water are periodically introduced into the first fermentor (1) and a neutral or alkaline liquid fraction withdrawn from the second fermentor (2) is periodically evacuated, this fraction substantially corresponding to the quantity of liquid introduced into the first fermentor (1) with each fresh batch of substrate.

5. Process according to any one of the preceding claims, characterised in that the contents of the first fermentor (1) are homogenized each time a fresh batch of organic substrate is introduced into said fermentor and the aforesaid mixed liquor is withdrawn.

6. Process according to any one of the preceding claims, characterised in that withdrawal of the excess of acids and/or alcohols from the first fermentor (1) is stopped during the introduction of each fresh batch of organic substrate into the first fermentor (1) and during each withdrawal of mixed liquor from this first fermentor (1).

7. Process according to any one of the preceding claims, characterised in that a container having a capacity greater than that of the container used as second fermentor (2) is used as first fermentor (1).

8. Process according to claim 7, characterised in that the ratio of the capacity of the first fermentor (1) to that of the second fermentor (2) is in the range of from 2.5 to 10.

9. Process according to claim 1 or 8, characterised in that the neutral or alkaline liquid effluent withdrawn from the second fermentor (2) is subjected to a decantation and the mass of methanogenic microorganisms separated by decantation is returned batchwise to the first fermentor (1).